# NEW EUROPEAN PATENT SPECIFICATION

(11) **EP 0 592 001 B2**
(45) Date of publication and mention of the opposition decision: **05.06.2002**
(45) Mention of the grant of the patent: 10.12.1997
(21) Application number: 93116357.0
(22) Date of filing: 08.10.1993
(51) Int. Cl.: A61F 13/15

(54) **Catamenial device with odor control**
Hygienische Einlage mit Geruchskontrolle
Dispositif catamenial avec contrôle des odeurs

(30) Priority: 09.10.1992 US 959013
(43) Date of publication of application: 13.04.1994
(73) Proprietor: Chicopee, Inc., North Charleston, SC 29406 (US)
(72) Inventor: Lasco, Vincent P., Mystic Islands, NJ 08087 (US)
(74) Representative: Groening, Hans Wilhelm, Dipl.-Ing.

(56) References cited:
- EP-A- 0 304 952
- EP-A- 0 347 746
- EP-A- 0 392 528
- WO-A-91/14414
- WO-A-91/15177
- DE-B- 1 239 059
- US-A- 2 690 415
- US-A- 4 289 513
- US-A- 4 748 065
- US-A- 5 122 407
- US-A- 5 154 960

## Description

This invention relates to absorbent products, such as catamenial devices, incontinence devices, disposable diapers and the like, which have an odor control material affixed to at least a portion of their outermost surface.

### Background of the Invention

A wide variety of absorbent structures designed not only to be efficient for the absorption of body fluids such as blood, urine, menses, and the like, but also to be sanitary and comfortable in use, are known in the literature. Disposable products of this type generally comprise some sort of fluid permeable topsheet or facing material, an absorbent core, and a fluid-impermeable backsheet material. Various shapes, sizes and thicknesses of such articles have been explored in an attempt to make their use more comfortable and convenient.

One aspect of sanitary protection products which has been under investigation for many years is that of odor control. Many body fluids have or develop an unpleasant odor when in contact with air and/or bacteria for prolonged periods. There have been several approaches to controlling this odor. One method has been to mask the odor with a pleasant scent such as perfume. Another approach has been the use of antimicrobial or bacteriostatic agents to control the bacterial or enzymatic reduction of components such as uric acid, urea, amino acids and the like found in urine and menstrual discharge, to ammonia and volatile amines which are the source of malodor.

Yet another approach has been absorbing malodors with odor-absorbing materials such as chlorophyll particles, activated carbon granules, ion exchange resins and the like.

The incorporation of particulate odor control materials in catamenial devices and the like has been accomplished by dispersing loose, individual particles on or into the absorbent core. Alternatively, a sheet in which or to which the particulate odor control material has been affixed is provided immediately beneath, within or on top of the absorbent core. Both these approaches have certain deficiencies. For example, if the particulate material is merely dispersed in or on the absorbent core, it has a tendency to "dust", or fall out of the absorbent core during shipping and handling or it may become undesirably concentrated in one section of the device. The incorporation of a sheet in which or to which odor control material has been affixed at a location beneath, within or on top of the absorbent core alleviates the dusting problem. However, this construction tends to remove at least a portion of the particulate odor control material from the regions of fluid impact and/or storage. Hence odors can be formed before the discharged fluid reaches the odor controlling material.

EP-A-304 952 discloses body fluid absorbent structures being usually composed of layers of materials, e.g. sanitary napkins and disposable diapers, which have a facing layer, a backing layer and an inside absorbent layer. A deodorant-containing water-swellable polymeric film-coated thin-flexible substrate made from nonwoven web, paper-tissue or water-insensitive film can be incorporated in any desired location in the body fluid absorbent structure. Thus, if a nonwoven web or impervious film is being used as part of the total sanitary napkin structure, it first can be coated with a hydrophilic water-insoluble water-swellable crosslinked polymeric film containing the powdered deodorant immobilized in said coating, and then used as before in the sanitary napkin construction. While the actual placement of the coated substrate in the absorbent product structure is not critical, it can be placed as close to the body as possible, e.g. on the body facing material, or immediately under the body facing layer.

EP-A-0 347 746 discloses an absorbent product according to the preamble of claim 1. In this document of prior art, it is stated that the adhesive, or other joining means, should not totally encapsulate or obscure the means for controlling odor and thereby prevent from acting on gaseous malodors. However, the layer providing odor control is always covered by, or embedded in, the cover sheet.

It is, therefore, an object of this invention to provide a sanitary protection, incontinence or the like product with an odor control mechanism that permits direct and essentially immediate contact between the discharged body fluid, such as menstrual fluid or urine, and the odor control material.

It is a further object of this invention to provide a sanitary protection or incontinence device with odor control properties which avoids the problems associated with "dusting" and undesirable concentration of odor control material in one section of the device .

Additional objects will becomes clear in the ensuing discussion describing the preferred embodiments of the products and processes of this invention.

This invention achieves the above-mentioned object by the features as disclosed in claim 1.

### Summary of the Invention

This invention relates to absorbent products, such as catamenial devices, incontinence devices, disposable diapers and the like which have an absorbent core capable of absorbing and holding liquid, said absorbent core being secured between a body-facing, fluid-permeable cover sheet and a garment-facing, fluid-impermeable backing sheet. As is well known, the absorbent core, the cover sheet and the backing sheet each have a first major, or upper, surface and a second major, or lower, surface. In each instance, the upper surface is that surface which, when the absorbent product is being used, faces the body of the wearer; the lower surface in each instance is that surface which faces away from the body of the user. Thus, in a typical disposable absorbent product, the lower surface of the absorbent core contacts the upper surface of the backing sheet, and the lower surface of the cover sheet contacts the upper surface of the absorbent core. As a result of this arrangement of structural parts, the upper surface of the facing material constitutes the upper surface of the absorbent product and is the surface of the product which contacts the body of the wearer when the device is in use. The lower surface of the backing sheet constitutes the lower surface of the absorbent product and is the surface of the device which is remote from the body when the device is in use. The lower surface of the backing sheet, when the absorbent product is a sanitary napkin, contacts an undergarment of the user and is typically provided with means, such as an adhesive, for securing the napkin to said undergarment. The body-facing, fluid-permeable cover sheet comprises an odor control material affixed to its body-facing, upper surface.

Preferably, the body-facing, fluid-permeable cover sheet is a nonwoven or woven fabric. The cover sheet comprises a discontinuous pattern of polymeric material on its upper surface and the polymeric material has an odor control material affixed thereto. When the cover sheet is properly positioned on the absorbent product, i.e., with the polymeric material/odor control material facing upwardly, the odor control material is positioned to be in direct contact with body fluid upon its discharge onto the absorbent product.

### Brief Description of the Drawings

Fig. 1 is a schematic, side elevational view of apparatus for making a liquid-permeable fabric comprising an odor control material on its upper surface;
Fig. 2 is an enlarged perspective of a fabric made in accordance with the present invention, said fabric comprising an odor control material on its upper surface;
Fig. 2A is a magnified view of the circled portion of Fig. 2;
Fig. 3 is a perspective of a sanitary napkin in accordance with the teachings of the present invention; and
Fig. 4 is a cross-section taken along lines 4-4 of Fig. 3.

### Detailed Description of the Preferred Embodiment

The absorbent products of this invention are generally catamenial devices, incontinence devices, or disposable diapers that have a body-facing, fluid-permeable nonwoven or woven fabric cover sheet, an absorbent core comprising hydrophilic and/or hydrophobic fibers for absorbing and retaining body fluid and a fluid-impermeable backing sheet on the garment-facing side of the absorbent core. The cover sheet comprises a coating of polymer material to which is affixed an odor control material.

The fluid-impermeable backing sheet is preferably a thin film of polyethylene on the order of one mil in thickness.

Preferably, the fluid-permeable cover sheet is a nonwoven fabric. As is well known in the art, the nonwoven fabric may be made entirely of hydrophilic fibers or entirely of hydrophobic fibers or it may be made of blends of hydrophilic and hydrophobic fibers. Hydrophilic fibers which may be used in the nonwoven fabric are, for example, cotton fibers, staple length rayon fibers and wood pulp fibers. Hydrophobic fibers which may be used in the nonwoven fabric are, for example, polyester fibers, polypropylene fibers, and bicomponent fibers having a polyester core and a polyethylene sheath. Typically, the fibers comprising the nonwoven fabric are unitized by the application thereto of a binder such as, for example, an acrylic polymer latex binder. In cases where the nonwoven fabric comprises thermoplastic fibers, the fabric may be unitized by application of heat which operates to fuse the fibers at their cross over points. In still other instances, the fibers comprising the nonwoven fabric may be held together by hydroentangling processes well known in the art.

The nonwoven fabric to which the odor control material is affixed is made by a process which includes the steps of:
a) providing a starting nonwoven fabric;
b) applying a polymeric material in a discontinuous pattern to at least one major surface of said nonwoven fabric while said polymeric material is in its wet or tacky state;
c) applying said odor control material to said wet or tacky polymer; and
d) causing said polymer to fuse, whereby said odor control material is affixed to the surface of said polymeric material.

As used herein, the term "discontinuous pattern of polymeric material" means that the polymeric material occupies only a fraction of the total area of the fabric surface to which it is being applied. For example, the polymer may be applied as a large number of tiny "globules" or "particles" which are spaced from each other in a regular pattern. These "globules" or "particles" of polymeric material may assume various shapes such as circular, oval, square, rectangular and the like. As another example, the "globules" or "particles" may be applied as a series of thin intersecting strands so as to form a random pattern on the surface of the fabric, or they may be applied so as to form an open, mesh-like pattern.

The polymer is most conveniently applied to a major surface of the nonwoven fabric in the form of a plastisol. A plastisol is a dispersion of a polymeric material in a plasticizer. Polymeric materials may include, for example, polyvinyl chloride and polyvinyl chloride copolymers. The preferred polymeric material is polyvinyl chloride. As is known in the art, a plasticizer is a nonvolatile organic liquid which comprises the continuous phase of the plastisol and which, when the plastisol has coalesced or fused into a solid material, functions to provide the polymeric material with increased softness and flexibility. Typical plasticizers include ethylene glycol derivatives, tricresyl phosphate, dibutyl phthalate, butyl benzyl phthalate and the like. Butyl benzyl phthalate, commercially available as Santicizer™ 160, is preferred for use in plastisols which contain polyvinyl chloride. A preferred plastisol for use in preparing fabric according to the present invention comprises polyvinyl chloride as the polymeric material and butyl benzyl phthalate as the plasticizer. In some instances, the plastisol may also contain a volatile organic solvent, in which case the resulting material is referred to as an "organosol".

The polymeric material may be conveniently applied to the surface of the nonwoven fabric by, for example, screen printing, rotogravure printing, spraying, casting or the like. The plastisol is preferably applied to the surface of the nonwoven substrate by screen printing.

The percentage of the total surface area of the nonwoven fabric covered by the polymeric material will vary depending upon the end use to which the fabric will be ultimately put. A male incontinence device, for example, may require more odor control material than a sanitary napkin and hence the former device will require a facing material which has a relatively larger amount of polymer and odor control material applied thereto. It will be understood, however, that the surface area of the nonwoven substrate covered by the polymeric material should be kept at a minimum, consistent with the amount of odor control material which is desired, in order not to unduly interfere with the passage of bodily fluids through the fabric.

Odor control materials useful in the practice of the present invention may be selected from any number of materials known for their ability to react with body fluids and reduce or control odors. For example, sodium bicarbonate, zeolites, ion exchange resins and activated charcoal may be used as odor control materials in the products of this invention.

The nonwoven fabric, after the polymeric material has been applied thereto in a discontinuous pattern and while said polymeric material is in a wet or tacky state, passes under a dispensing unit where the odor control material is dispensed onto the upper surface of the web, and adhered to the surface of the tacky polymeric material. Those portions of the odor control material which do not come into contact with and adhere to the polymeric material are removed from nonwoven fabric 12. This can be accomplished by, e.g., passing the nonwoven fabric over a vacuum slot located between dispenser unit 5 and fusing apparatus 7 or between apparatus 7 and take-up roll 9. The nonwoven fabric having the polymeric material and odor control material on the upper surface thereof is then subjected to a fusing or curing step in which the liquid plastisol in its wet or tacky state is converted to a solid. The conversion of a plastisol from its initially liquid state to its final solid form is variously referred to in the art as curing, coalescing or fusing. When used in this specification and the appended claims, the term "fusing" includes the terms "curing" and "coalescing". The fusing of the plastisol is accomplished by the application of heat thereto. The heat can be supplied by, for example, microwave devices, infrared devices, conventional gas-fired or electric ovens and the like. Preferably, the energy needed for fusing the plastisol is provided by short wavelength infrared lamps.

Preferably, an opacifying agent is added to the plastisol prior to its application to the fabric. Suitable opacifiers are clays, calcium carbonate, titanium dioxide and the like. Titanium dioxide is the preferred opacifier.

The advantages of the products of this invention are several. The anchoring of the odor control material in the polymeric material substantially eliminates problems associated with migration and dusting. Furthermore, the presence of the odor control material on the upper surface of the fabric ensures contact between the odor control material and the body fluid as it is deposited on the absorbent product. Another advantage of the process and products of this invention is that the odor control material is maintained at a location remote from the absorbent core of the product. This substantially reduces the possibility that the odor control material will come into contact with the fibrous materials, e.g., wood-pulp, usually employed in the absorbent core and, accordingly, greatly reduces the possibility on any adverse reaction, e.g., yellowing of fibers, which might otherwise be caused by contact of the fibrous materials with the odor control material. The use of a polymeric material which is thermoplastic also permits the heat sealing of the product to ensure a sturdier construction. When, as is preferable, the polymeric material is applied to the fabric in the form of a plastisol, which is a nonaqueous material, the possibility of an adverse reaction between water and the odor control material is greatly reduced. For example, when a plastisol is used to provide the polymeric material and the odor control material is sodium bicarbonate, the possibility of any adverse reaction involving water and the sodium bicarbonate is virtually eliminated.

The following example is illustrative of the process and products of this invention. However, this example is merely illustrative and does not serve to limit the scope of the invention.

### Example 1

A starting nonwoven fabric is prepared as follows:

A fibrous card web weighing about 11,6 g/m² (214 grains per square yard) and comprising 100% extra dull bleached rayon fibers, 27 tex (3 denier) and 2,54 - 1,43 cm (1-9/16 inches) in length, is intermittently bonded by a rotogravure process using an engraved printing roll having 4 horizontal wavy lines per 2,54 cm (1 inch), as measured in the machine or long direction. The width of each line as measured peripherally on the engraved printing roll is 0,048 cm (0.019 inch). The binder resin in an N-methylolacrylamide modified, crosslinkable copolymer of ethyl acrylate and butyl acrylate. The aqueous binder composition comprises 4,08 kg (9 pounds) of a 50% solids aqueous dispersion of the binder resin (actual weight of resin 2,04 kg (4.5 pounds)), 0,63 kg (1.4 pounds) of a conventional thickening agent, 0,023 kg (0.05 pounds) of a conventional antifoam agent and small amounts of conventional pigments, anti-oxidants, etc. The fibrous card web is wetted with water. The binder is then applied to the wet web and drying takes place on heated drying cans at a temperature of about 132,2°C (270°F). The finished dry weight of the bonded nonwoven fabric is about 14,1 g/m² (260 grains per square yard).

A liquid plastisol was prepared by combining 45 parts by weight of Geon™ 185, 45 parts by weight of Santicizer™ 160 and 10 parts by weight of titanium dioxide. Geon™ 185 is a polyvinyl chloride resin in powder form commercially available from B. F. Goodrich & Company. Santicizer™ 160 is butyl benzyl phthalate and is commercially available from Monsanto Chemical Company. The above-mentioned three-component mixture was stirred in a Hobart Mixture for approximately 2 hours at room temperature to provide a plastisol suitable for use in the practice of the present invention.

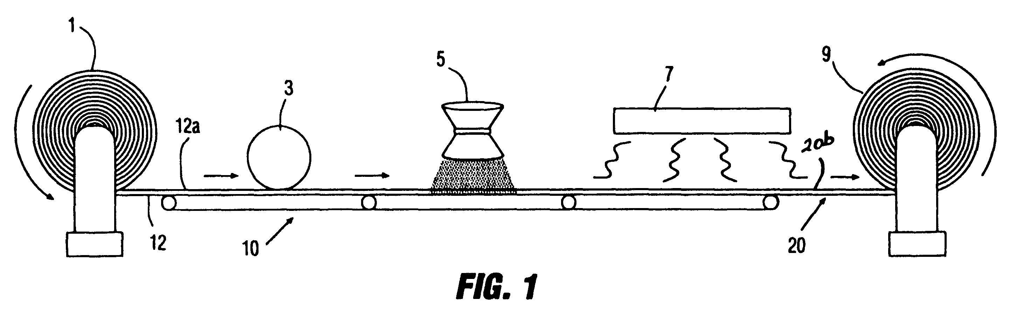

Referring now to Figure 1 of the drawings, the above-described starting nonwoven fabric 12 was fed from let-off roll 1 onto conveyor belt 10. Nonwoven fabric 12 passed under screen printing unit 3 to which was supplied the aforementioned plastisol. The screen printing unit which was used was a Kraemer Koater which was obtained from Kraemer Koating Company of Toms River, New Jersey. The print screen in the Kraemer Koater comprised a series of circular openings arranged in a straight line pattern. The openings had a diameter of 0,114 cm (0.045 inch) and there were 34,9 openings/cm² (225 openings/in²). The open area of the print screen was about 36%. The screen printing unit deposited a pattern of tiny dots of the above-mentioned plastisol on the upper surface 12a of nonwoven fabric 12. The fabric with the wet plastisol applied thereto then passed under powder dispenser unit 5 where fine particles of sodium bicarbonate were directed through a 40 mesh screen and deposited on the upper surface of the nonwoven fabric and were adhered to the wet plastisol. The sodium bicarbonate was obtained from Fisher Scientific Company. The nonwoven fabric was then passed under an infrared curing apparatus 7 comprising a bank of six (6) short wavelength infrared lamps. Each such lamp was approximately 55,9 cm (22 inches) in length and 11 millimeters in diameter. The peak wave length emission range of the lamps was 0.75-1.3 microns. The infrared lamps were operated at approximately 460 volts and the lamps themselves were located approximately 27,94 cm (11 inches) above the upper surface of the web. The web was conducted beneath the infrared curing apparatus at a speed of approximately 50,8 cm/m/n (20 inches per minute). The final fabric 20 was then wound up on take-up roll 9 to await further use. The upper surface 20b of the final nonwoven fabric 20 comprised a discontinuous pattern of polymeric material with the sodium bicarbonate odor control material affixed thereto.

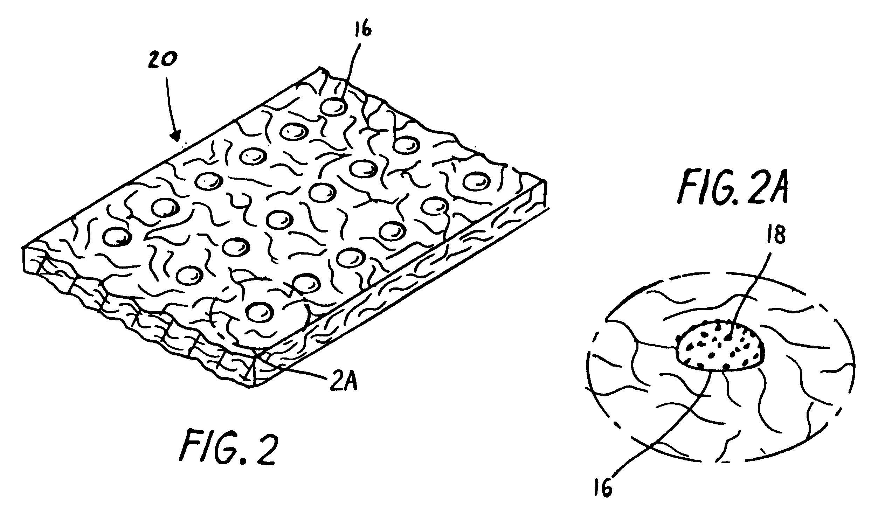

As seen in Figs. 2 and 2A, nonwoven fabric 20 comprises discrete "particles" or "globules" 16 of polymeric material 16 to which is affixed odor control material 18.

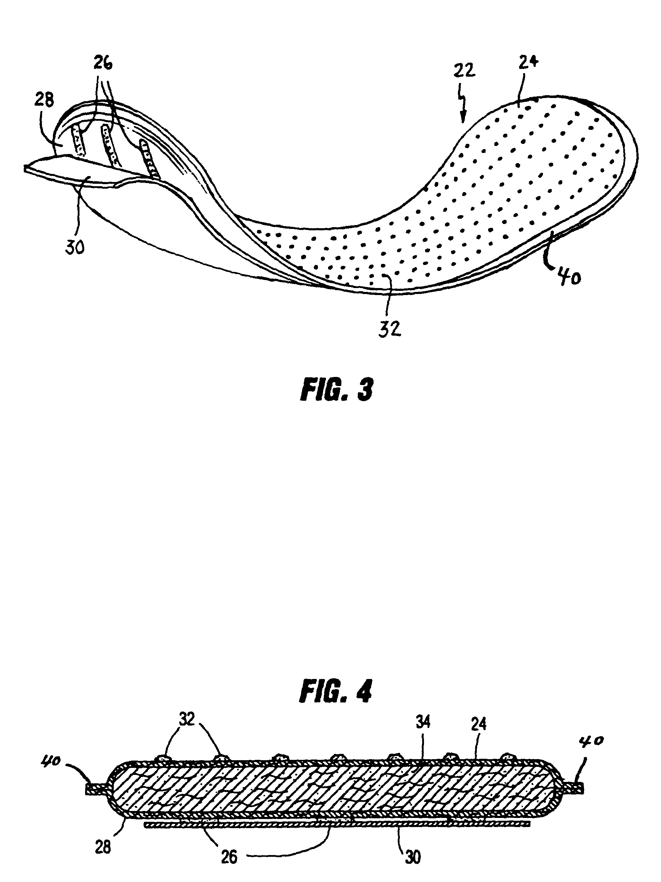

Referring to Figs. 3 and 4 of the accompanying drawings, there is illustrated an absorbent product in the form of a sanitary napkin in accordance with the teachings of the present invention. Sanitary napkin 22 comprises a garment-facing, fluid-impermeable backing sheet 28, an absorbent core 34 and a body-facing, fluid-permeable cover sheet 24 made as described in Example 1. As seen in Fig. 4, absorbent core 34 is placed between backing sheet 28 and facing sheet 24. The backing sheet and facing sheet are secured to each other, e.g., with an adhesive, at the periphery 40 of the napkin. The upper surface of cover sheet 24 comprises small dots 32 of polymer which have the odor control material, i.e., sodium bicarbonate, affixed thereto. Three lines of adhesive 26 are disposed on the lower surface of the backing sheet to provide means for adhering the napkin to the crotch portion of an undergarment. The adhesive lines 26 are protected prior to use by being covered with a strip of release paper 30. It will be apparent that the tiny dots of polymer with the odor control material affixed to the exposed surfaces thereof will contact the body when the napkin is being used and menstrual fluid, upon discharge onto the upper surface of the napkin, will substantially immediately come into contact with the odor control material.

## Claims

1. An absorbent product comprising an absorbent core (34), a liquid-impermeable backing sheet (28) and a liquid-permeable cover sheet (24), said absorbent core (34) being secured between said backing sheet (28) and said cover sheet (24), said cover sheet (24) comprising a fabric having a first, upper, surface constituting the outer surface of said absorbent product facing the body of the wearer and a discontinuous pattern of polymeric material (16), said polymeric material having an odor control material (18) affixed thereto, **characterized in that** the polymeric material (16) is arranged on the first, upper, surface of the cover sheet (24).

2. The absorbent product of claim 1 wherein said polymeric material (16) comprises a polymer selected from the group consisting of polyvinyl chloride and polyvinyl chloride copolymers.

3. The absorbent product of claim 1 wherein said polymeric material (16) comprises polyvinyl chloride.

4. The absorbent product of claim 1 wherein said polymeric material (16) is arranged in a pattern of tiny dots.

5. The absorbent product of claim 1 wherein said odor control material (18) is selected from the group consisting of sodium bicarbonate, zeolites, ion exchange resins and activated charcoal.

6. The absorbent product of claim 1 wherein said odor control material (18) comprises sodium bicarbonate.

7. The absorbent product of claim 1 wherein said polymeric material (16) is derived from a plastisol.

8. The absorbent product of claim 1 wherein said plastisol comprises polyvinyl chloride.

9. The absorbent product of claim 1 wherein said cover sheet is a nonwoven fabric; said polymeric material (16) is derived from a plastisol comprising polyvinyl chloride and is arranged on said first, upper, surface in a discontinuous pattern; and said odor control material (18) is sodium bicarbonate.

## Patentansprüche

1. Absorbierendes Produkt, enthaltend einen absorbierenden Kern (34), eine flüssigkeitsundurchlässige Rückschicht (28) und eine flüssigkeitsdurchlässige Deckschicht (24), wobei der absorbierende Kern (34) zwischen der Rückschicht (28) und der Deckschicht (24) befestigt ist, die Deckschicht (24) einen textilen Werkstoff mit einer ersten, oberen Fläche, die die äußere dem Körper des Trägers zugekehrte Oberfläche des absorbierenden Produkts bildet und ein diskontinuierliches Muster aus polymerem Material umfaßt, wobei das polymere Material ein Geruchsbekämpfungsmittel (18) aufweist, das daran angebracht ist, **dadurch gekennzeichnet, dass** das polymere Material (16) auf der ersten, oberen Fläche der Deckschicht (24) angeordnet ist.

2. Absorbierendes Produkt nach Anspruch 1, wobei das polymere Material (16) ein Polymeres aus der Gruppe Polyvinylchlorid und Polyvinylchlorid-Copolymere umfaßt.

3. Absorbierendes Produkt nach Anspruch 1, wobei das polymere Material (16) Polyvinylchlorid umfaßt.

4. Absorbierendes Produkt nach Anspruch 1, wobei das polymere Material (16) in einem Muster von feinen Punkten angeordnet ist.

5. Absorbierendes Produkt nach Anspruch 1, wobei das Geruchsbekämpfungsmittel (18) aus der Gruppe Natriumcarbonat, Zeolithe, Ionenaustauscherharze und Aktivkohle ausgewählt ist.

6. Absorbierendes Produkt nach Anspruch 1, wobei das Geruchsbekämpfungsmaterial (18) Natriumcarbonat umfaßt.

7. Absorbierendes Produkt nach Anspruch 1, wobei das polymere Material (16) von einem Plastisol abgeleitet ist.

8. Absorbierendes Produkt nach Anspruch 1, wobei das Plastisol Polyvinylchlorid umfaßt.

9. Absorbierendes Produkt nach Anspruch 1, wobei die Deckschicht ein Faservlies ist, das polymere Material (16) von einem Plastisol, das Polyvinylchlorid umfaßt, abgeleitet ist und auf der ersten, oberen Oberfläche in einem diskontinuierlichen Muster angeordnet ist; und das Geruchsbekämpfungsmaterial (18) Natriumcarbonat ist.

## Revendications

1. Produit absorbant comprenant une partie centrale absorbante (34), une feuille de dessous imperméable aux liquides (28) et une feuille de dessus perméable aux liquides (24), ladite partie centrale absorbante (34) étant fixée entre ladite feuille de dessous (28) et ladite feuille de dessus (24), ladite feuille de dessus (24) comprenant un tissu ayant une première surface supérieure constituant la surface externe dudit produit absorbant faisant face au corp de l'utilisateur, et un motif discontinu de matériau polymère (16), ledit matériau polymère de contrôle des odeurs (18) fixé dessus, **caractérisé en ce que** le matériau polymère (16) est disposé sur la première surface supérieure de la feuille de dessus (24).

2. Produit absorbant selon la revendication 1, dans lequel ledit matériau polymère (16) comprend un polymère sélectionné à partir du groupe composé de chlorure de polyvinyle et de copolymère de chlorure de polyvinyle.

3. Produit absorbant selon la revendication 1, dans lequel ledit matériau polymère (16) comprend du chlorure de polyvinyle.

4. Produit absorbant selon la revendication 1, dans lequcl ledit matériau polymère (16) est disposé selon un motif de petits points.

5. Produit absorbant selon la revendication 1, dans lequel ledit matériau de contrôle des odeurs (18) est sélectionné à partir du groupe constitué du bicarbonate de sodium, des zéolites, des résines à échange d'ion et du charbon de bois activé.

6. Produit absorbant selon la revendication 1, dans lequel ledit matériau polymère (18) comprend du bicarbonate de sodium.

7. Produit absorbant selon la revendication 1, dans lequel ledit matériau polymère (16) est dérivé à partir d'un plastisol.

8. Produit absorbant selon la revendication 1, dans lequel ledit plastisol comprend du chlorure de polyvinyle.

9. Produit absorbant selon la revendication 1, dans lequel ladite feuille de dessus est un tissu non tissé ; ledit matériau polymère (16) est dérivé d'un plastisol comprenant du chlorure de polyvinyle et est disposé sur ladite première surface supérieure sous la forme d'un motif discontinu ; et ledit matériau de contrôlc des odeurs (18) est du bicarbonate de sodium.
